# EUROPÄISCHE PATENTSCHRIFT

(11) **EP 0 580 599 B1**
(45) Veröffentlichungstag und Bekanntmachung des Hinweises auf die Patenterteilung: **19.01.2000**
(21) Anmeldenummer: 92905633.1
(22) Anmeldetag: 06.03.1992
(51) Int. Cl.: C08G 18/48, C08J 9/02, A61L 15/26

(54) **HYDROPHILE SCHÄUME UND VERFAHREN ZU DEREN HERSTELLUNG**
HYDROPHILIC FOAMS AND PROCESS FOR PRODUCING THEM
MOUSSES HYDROPHILES ET LEUR PROCEDE DE PRODUCTION

(30) Priorität: 05.04.1991 DE 4111098
(43) Veröffentlichungstag der Anmeldung: 02.02.1994
(73) Patentinhaber: Beiersdorf Aktiengesellschaft, 20245 Hamburg (DE)
(72) Erfinder: NASS, Martina, D-2103 Hamburg 95 (DE); SACKER, Heidi, D-2000 Hamburg 20 (DE)
(74) Vertreter: Dinné, Erlend
(86) Internationale Anmeldenummer: DE9200192
(87) Internationale Veröffentlichungsnummer: WO9217518

(56) Entgegenhaltungen:
- EP-A- 0 196 364
- EP-A- 0 271 292
- EP-A- 0 299 122
- US-A- 3 978 855

## Beschreibung

Die vorliegende Erfindung betrifft Hydrogelschäume, insbesondere solche für medizinische Anwendungen, sowie Verfahren für ihre Herstellung.

Hydrogele sind makromolekulare, natürliche oder synthetische Stoffe die aufgrund eines hohen Gehaltes an hydrophilen Gruppen in der Lage sind, Wasser absorptiv zu binden. Die Wasseraufnahmekapazität vieler Hydrogele beträgt das Mehrfache des Eigengewichtes der wasserfreien Substanz.

Hydrogele werden in vielfältiger Form in der Medizin eingesetzt. Besonders geeignet sind sie in der Wundversorgung; sie können
* Wunden vor der Austrocknung schützen
* Wundsekret aufsaugen
* als Matrix für Wirkstoffe aller Art dienen
* als Basis für die Besiedelung mit autologen oder heterologen Hautzellen dienen

Hydrogele können unter anderem in Form von Schäumen verwendet werden.

Schäume zur Versorgung von Hautwunden oder chirurgischen Wunden sind an sich bekannt. Hauptsächlich finden dabei Polyurethanschäurne oder Kollagenschäume Verwendung.

Die Hydrogele des Standes der Technik haben jedoch verschiedene Nachteile:

Aufgrund ihrer Hydrophilie sind die meisten in Frage kommenden Stoffe wasserlöslich. Dies ist meist unerwünscht, weil derartige Produkte nicht formstabil sind. Außerdem lösen sich derartige Produkte in unerwünschter Weise am Einsatzort auf und stehen dann für den vorgesehenen Zweck nicht mehr zur Verfügung.

Andere Produkte zeichnen sich durch starke Polymervernetzung aus. Dadurch werden zwar manche der Nachteile der vorgenannten Stoffklasse vermieden. Die Quellbarkeit dieser Stoffe ist jedoch weitgehend eingeschränkt oder verloren. Überdies sind die synthetischen Vernetzer, die hier eingesetzt werden, alle mehr oder weniger toxisch.

Auch selbstklebende Geischäume sind an sich bekannt. Diese lassen sich zwar im allgemeinen recht gut auf der Haut fixieren, haben aber meistens den Nachteil, daß ihre Wasseraufnahme- und -abgabekapazität stark eingeschränkt sind.

In EP-A-0 097 846 werden Wundbehandlungsmittel auf der Basis von Hydrogelen beschrieben. Dabei wird Gelatine in fester Form als Pulver, Schuppen oder Blatt in einer Zwei-Phasen Reaktion mit Vernetzern wie Formaldehyd, Glyoxal, Glutarsäuredialdehyd, Dicarbonsäurechloriden und/oder Diisocyanaten umgesetzt.

Der Vernetzer wirkt dabei auf die gequollene, nicht gelöste Gelatine ein. Dieses Verfahren und die daraus erhaltenen Produkte sind mit erheblichen Nachteilen behaftet, da die verwendeten Vernetzer erhebliche Zellschädigungen bewirken können.

Zudem ist das Verfahren nicht oder nur schwer reproduzierbar. Die Vernetzung hängt nicht nur von der Konzentration der verwendeten Vernetzer ab, sondern auch von Parametern wie Temperatur und Einwirkzeit der Reaktanden. Ferner sind die effektive Oberfläche und das mittlere Molekulargewicht der handelsüblichen Gelatinearten deutlichen Schwankungen unterworfen, so daß auch die Eigenschaften des vernetzten Hydrogels schwer voraussehbar sind.

Weiterhin ist in EP-A-0 097 846 ein Verbandmaterial aus Hydrogelen auf Polyvinylakoholbasis beschrieben. Als Vernetzer wird Formaldehyd eingesetzt, der, wie eingangs erwähnt, physiologisch bedenklich ist.

Auch Schäume aus Polyvinylalkohol oder Collagen sind bekannt und gebräuchlich. Da deren Matrixsubstanzen aber die voranstehend beschriebenen Nachteile haben, sind sie zur Wundversorgung allenfalls bedingt geeignet.

Weiterhin sind hydrophile Schäume aus Polyurethangelen bekannt. Die PCT-Patentanmeldungsschrift WO-88/01878 beschreibt selbstklebende Polyurethanschäume bzw. Polyurethanschaumgele, welche unter anderem Methacrylate enthalten können.

Polyurethangele auf der Basis einer Polyurethanmatrix und höhermolekularen Polyolen werden auch in EP-B-0 057 839 beschrieben. Selbsthaftende Flächengebilde aus Polyurethangelen sind aus EP-B-0 147 588 bekannt. Die in diesen beiden letztgenannten Schriften offenbarten Polyurethangele sind jedoch wasserfrei und ungeschäumt.

Es war daher die Aufgabe der Erfindung, Hydrogelschäume zu entwickeln, welche nicht die Nachteile des Standes der Technik haben und als Wundversorgungsmittel geeignet sind. Außerdem sollten die Schäume nach reproduzierbaren Verfahren wirtschaftlich herstellbar sein.

Überraschend wurde gefunden, und darin besteht die Lösung dieser Aufgaben, daß

Hydrophile Schäume, basierend auf einer Polyurethangelmasse, die erhältlich ist aus
- einem oder mehreren Polyetherpolyolen, gewählt aus der Gruppe der durch Anlagerung von Propylenoxid und gegebenenfalls Ethylenoxid an übliche Startermolekülegebildeten Substanzen
- einem oder mehreren Diisocyanaten,
- einem oder mehreren Beschleunigem, gewählt aus der Gruppe der üblichen Beschleuniger für Polyurethane,
- wobei das Gewichtsverhältnis von Polyetherpolyol : Diisocyanat im Bereich von 15 : 1 bis 5 : 1 gewählt wird,
- einem oder mehreren Superabsorbem, wobei diese Superabsorber gewählt werden aus der Gruppe der Natriumsalze der Polyacrylate oder aus der Gruppe der Carboxymethylcellulosederivate, Karaya, Cellulose, Pektin, Polyvinylalkohol, Polyvinylpyrrolidon und Gelatine
- Wasser,
- sowie gegebenenfalls üblichen Hilfs- und/oder Zusatzstoffen
   die Nachteile des Standes der Technik beseitigen.

In besonderem Maße zeichnen sich die erfindungsgemäßen hydrophilen Schäume durch eine extrem hohe Wasseraufnahmekapazität aus. Die Wasseraufnahmefähigkeit kann beispielsweise das Hunderfache des Trockeneigengewichtes der erfindungsgemäßen Hydrogelschäume betragen.

Zwar beschreibt die EP-A 0 196 364 ein Herstellungsverfahren für flexible hydrophile Schäume auf Polyurethanbasis. Diese Schrift konnte jedoch nicht den Weg zur vorliegenden Erfindung ebnen.

Die Startermoleküle für die Polyetherpolyole sind bevorzugt Pentaerythrit, Sorbit, Trimethylolpropan oder Ethylendiamin.

Besonders vorteilhaft werden solche Polyetherpolyole eingesetzt, wie sie auch in EP-B-0 057 839 und EP-B-0 147 588 beschrieben werden.

Die Diisocyanate werden entweder gewählt aus der Gruppe der unmodifizierten aromatischen oder aliphatischen Diisocyanate, sehr vorteilhaft beispielsweise 4,4'-Diisocyanatodiphenylmethan, oder aber aus durch Präpolymerisierung mit Polyolen oder Polyetherpolyolen gebildete modifizierte Produkte. Als sehr günstig hat sich beispielsweise durch Präpolymerisierung mit Tripropylenglycol verflüssigtes 4,4'-Diisocyanatodiphenylmethan erwiesen. Besonders vorteilhaft werden solche Diisocanate eingesetzt, wie sie auch in EP-B-0 057 839 und EP-B-0 147 588 beschrieben werden.

Besonders vorteilhaft werden die Beschleuniger gewählt aus der Gruppe bestehend aus
- Organischen Säuren, insbesondere p-Toluolsulfonsäure, n-Butylphosphorsäure,
- Organozinnverbindungen, einschließlich deren Salze organischer und anorganischer Säuren, insbesondere Zinn-Naphthenat, Zinnbenzoat, Dibutylzinndioctoat, Dibutylzinndilaurat, Zinn-II-ethylhexoat und Dibutylzinndiacetat,
- Eisensalzen höherer Fettsäuren, insbesondere Eisenstearat,
- Isophorondiamin, Methylendianilin, Imidazolen.
- tertiären Aminen, insbesondere der Trialkylamine, wobei die Alkylreste vorteilhaft 2 - 6 Kohlenstoffatome haben.

Unter Superabsorbem im Sinne der vorliegenden Erfindung werden insbesondere verstanden Substanzen, die ein hohes Absorptionsvermögen für Flüssigkeiten, namentlich Wasser, aufweisen und die aufgenommene Flüssigkeiten auch unter Belastung festhalten, im Gegensatze zu beispielsweise Zellstoffasem.

Die Superabsorber werden gewählt aus der Gruppe der Natriumsalze der Polyacrylate, besonders vorteilhaft werden solche gewählt, die unvernetzt oder nur schwach vernetzt sind und aus allen üblichen Handelsprodukten dieser Art gewählt werden können. Insbesondere sind solche Produkte geeignet wie sie in der DE-A-37 13 601 offenbart werden. Aber auch andere übliche Superabsorber, z.B. gewählt aus der Gruppe der Carboxymethylcellulosederivate, Karaya, Cellulose, Pektin, Polyvinylalkohol, Polyvinylpyrrolidon oder Gelatine sind Gegenstand der Erfindung.

Die Hilfs- und Zusatzstoffe können ebenfalls aus den üblichen Substanzklassen gewählt werden. Insbesondere sind vorteilhaft Farbstoffe, Pigmente, Lichtschutzmittel, Konservierungsmittel, Duftstoffe, Antimikrobiell wirksame Substanzen, sonstige Wirkstoffe wie beispielsweise kühlend wirkende Substanzen (z.B.Menthol) oder solche die die Durchblutung fördern oder ein Wärmegefühl erzeugen und so weiter.

Besonders vorteilhaft können die erfindungsgemäßen Hydrogelschäume nach an sich bekannten Verfahren auf flächige Träger, z.B. Gewebe, Gewirke, Vliese, Folien oder dergleichen aufgetragen werden.

Erfindungsgemäß ist ferner auch ein Verfahren zur Herstellung hydrophiler Schäume, dadurch gekennzeichnet, daß
- ein oder mehrere Polyetherpolyole, gewählt aus der Gruppe der durch Anlagerung von Propylenoxid und gegebenenfalls Ethylenoxid an übliche Startermoleküle gebildeten Substanzen
- ein oder mehrere Diisocyanate,
- ein oder mehrere Superabsorber
- ein oder mehrere Beschleuniger, gewählt aus der Gruppe der üblichen Beschleuniger für Polyurethane,
- Wasser,
   vereinigt und miteinander vermischt und gegebenenfalls zusätzlich mechanisch und/oder durch Gaseinleitung zu Schäumen aufgeschlagen werden und gegebenenfalls
- zu flächigen Gegenständen ausgegossen oder ausgestrichen werden oder
- auf flächige Träger aufgebracht werden oder
- zu raumfüllenden Gegenständen gegossen werden.

Die Polyetherpolyole können beispielsweise Handelsprodukte sein wie Levagel VP SN 100. Als Diisocyanate haben sich Handelsprodukte wie Desmodur PF, Desmodur N 100, IPDI und Desmodur W als geeignet erwiesen. Als Superabsorber hat sich Natrium-polyacrylat insbesondere ein Natrium-polyacrylathat der Bezeichnung Favor 922-SK als vorteilhaft herausgestellt.

Für die Beschleuniger, so das Dibutylzinndilaurat, ist Desmorapid Z/SN, für Zinn-II-ethylhexoat Desmorapid SO ein vorteilhaftes Handelsprodukt.

Vorteilhaft sind die erfindungsgemäßen Schäume erhältlich aus
- mindestens 20 Gew.-%: Polyetherpolyol
- *: Diisocyanat in einer Menge, daß das Gewichtsverhältnis Polyetherpolyol : Diisocyanat von 15 : 1 bis 5 : 1 eingehalten wird
- * 5 - 60 Gew.-%: Superabsorber
- * 0,001 - 1,00 Gew.-%: Beschleuniger
- * 0,01 - 20 Gew.-%: Wasser

Besonders vorteilhaft sind die erfindungsgemäßen Schäume erhältlich aus
- mindestens 20 Gew.-%: Polyetherpolyol
- *: Diisocyanat in einer Menge, daß das Gewichtsverhältnis Polyetherpolyol : Diisocyanat von 15 : 1 bis 5 : 1 eingehalten wird
- * 5 - 60 Gew.-%: Na-Polyacrylat
- * 0,001 - 1,00 Gew.-%: Beschleuniger
- * 0,01 - 20 Gew.-%: Wasser

Bevorzugt sind die erfindungsgemäßen Schäume erhältlich aus
- * 50 - 70 Gew.-%: Polyetherpolyol
- mindestens 5 Gew.-%: Diisocyanat mit der Maßgabe, daß das Gewichtsverhältnis Polyetherpolyol : Diisocyanat von 15 : 1 bis 5 : 1 eingehalten wird
- * 15 - 35 Gew.-%: Superabsorber
- * 0,05 - 1,00 Gew.-%: Beschleuniger
- * 0,1 - 10 Gew.-%: Wasser

Besonders bevorzugt sind die erfindungsgemäßen Schäume erhältlich aus
- * 50 - 70 Gew.-%: Polyetherpolyol
- mindestens 5 Gew.-%: Diisocyanat mit der Maßgabe, daß das Gewichtsverhältnis Polyetherpolyol : Diisocyanat von 15 : 1 bis 5 : 1 eingehalten wird
- * 15 - 35 Gew.-%: Na-Polyacrylat
- * 0,05 - 1,00 Gew.-%: Beschleuniger
- * 0,1 - 10 Gew.-%: Wasser

Vorteilhaft ist es insbesondere, Verhältnisse von Polyetherpolyol : Diisocyanat von etwa (8 bis 12) : 1 zu wählen.

Es ist möglich, die Hafteigenschaften der Erfindungsgemäßen Schäume durch das Verhältnis von Polyol zu Isocyanat zu steuern. So erhält man bei einem Verhältnis von Polyol : Isocyanat von etwa 10 : 1 schwach, wenn überhaupt, adhäsive und kräftig kohäsive Schäume. Wird das Verhältnis größer als etwa 10 : 1, so wird die Adhäsionskraft größer. Wird das Verhältnis geringer, so nimmt auch die Kohäsionskraft ab.

Die Schäume können nach einer vorteilhaft etwa 2 - 30 Minuten messenden Topfzeit zu flächigen Gebilden ausgegossen oder ausgestrichen werden. Auf diese Weise sind Schaumdicken von 0,10 mm bis mindestens 15 mm problemlos erreichbar. Es ist aber auch möglich und vorteilhaft, Gegenstände aus den erfindungsgemäßen Hydrogelschäumen anzufertigen, welche nicht flächig, sondern ausgeprägt raumfüllend sind, beispielsweise durch übliche Gußverfahren.

Die entstehenden Schäume sind offenporige Schäume, wenn der Schaumrohstoff auf einen abhäsiven Träger, also einen Träger, dessen Oberfläche klebstoffabweisend ausgebildet ist, gegossen und abgedeckt werden. Es ist dann also nicht notwendig, sie durch Schnitt in offenporige Schäume umzuwandeln.

Obwohl es, wie erwähnt, möglich ist, die Hafteigenschaften der erfindungsgemäßen Schäume zu steuern, so daß die erhaltenen Schäume oder die damit beschichteten Träger selbstklebend sein können, können sie vorteilhaft zudem auf einer oder der beiden oder auch beiden Großflächen mit einer üblichen Selbstklebemasse beschichtet werden.

Der fertige Schaum, vorzugsweise die vorabbeschriebenen flächigen Gebilde, vorteilhaft aber auch die mit Hydrogelschaum beschichteten Träger, können beispielsweise dazu dienen, Oberflächenwunden oder chirurgische Wunden zu versorgen.

Ein besonderer überraschender Vorteil der erfindungsgemäßen Schäume absolut rückstandsfrei vom menschlichen Gewebe, und insbesondere von Wunden wieder entfernt werden kann, ohne daß das ohnehin beeinträchtigte Gewebe weitere Schäden erfährt.

Ein besonders vorteilhaftes Anwendungsgebiet ist daher die Versorgung tiefer Wunden. Insbesondere deshalb, weil die erfindungsgemäßen Schäume aufeinander haften können.

Mehrere dünne Schaumschichten, die einzeln sehr bequem in geeignete Formen zu schneiden sind können so in oder auf der Wunde übereinandergestapelt werden, ohne daß es weiterer Fixierungshilfsmittel bedürfte. Dadurch wird die gleiche Wirkung erzielt wie mit einer dicken Schaumschicht.

Die kohäsive Eigenschaft einiger der erfindungsgemäßen Schäume eignet sie auch gut zur Verwendung als kohäsive Binden. Darunter sind solche Binden zuverstehen, welche zwar auf sich selbst, nicht jedoch auf menschlichem Gewebe haften.

Die nachfolgenden Beispiele sollen die Erfindung erläutern, ohne daß aber beabsichtigt ist, die Erfindung auf diese Beispiele zu beschränken.

### Beispiel 1

| | |
|---|---|
| Levagel VP SN 100 | 61 Gew.-% |
| Favor 922 SK | 24 Gew.-% |
| Dibutylzinndilaurat | 0,1 Gew.-% |
| Wasser | 4,9 Gew.-% |
| Desmodur PF | 7 Gew.-% |

### Beispiel 2

| | |
|---|---|
| Levagel VP SN 100 | 69 Gew.-% |
| Favor 922 SK | 18 Gew.-% |
| Dibutylzinndilaurat | 0,1 Gew.-% |
| Wasser | 2,9 Gew.-% |
| Desmodur PF | 10 Gew.-% |

### Beispiel 3

| | |
|---|---|
| Levagel VP SN 100 | 58 Gew.-% |
| Favor 922 SK | 33 Gew.-% |
| Dibutylzinndilaurat | 0,1 Gew.-% |
| Wasser | 2,9 Gew.-% |
| Desmodur PF | 6 Gew.-% |

Die Zusammensetzungen gemäß den Beispielen 1 - 3 werden auf einmal zusammengegeben und miteinander vermischt. Die Topfzeit, d.i. die Verarbeitungszeit der noch flüssigen Mischung, beträgt 1,5 - 10 Minuten. Die Schäume können ausgestrichen und weiter verarbeitet werden.

## Patentansprüche

1. Hydrophile Schäume, basierend auf einer Polyurethangelmasse, die erhältlich ist aus
- einem oder mehreren Polyetherpolyolen, gewählt aus der Gruppe der durch Anlagerung von Propylenoxid und gegebenenfalls Ethylenoxid an übliche Startermolekülegebildeten Substanzen
- einem oder mehreren Diisocyanaten,
- einem oder mehreren Beschleunigern, gewählt aus der Gruppe der üblichen Beschleuniger für Polyurethane,
- wobei das Gewichtsverhältnis von Polyetherpolyol : Diisocyanat im Bereich von 15 : 1 bis 5 : 1 gewählt wird,
- einem oder mehreren Superabsorbem, wobei diese Superabsorber gewählt werden aus der Gruppe der Natriumsalze der Polyacrylate oder aus der Gruppe der Carboxymethylcellulosederivate, Karaya, Cellulose, Pektin, Polyvinylalkohol, Polyvinylpyrrolidon und Gelatine
- Wasser,
- sowie gegebenenfalls üblichen Hilfs- und/oder Zusatzstoffen.

2. Schäume nach Anspruch 1, dadurch gekennzeichnet, daß das Verhältnis von Polyetherpolyol: Diisocyanat im Bereich von 12 : 1 bis 8 : 1 gewählt wird

3. Schäume nach Anspruch 1, dadurch gekennzeichnet, daß die Polyetherpolyole gewählt werden aus solchen, hergestellt mit Pentaerythrit, Sorbit, Trimethylolpropan oder Ethylendiamin als Startermolekülen.

4. Schäume nach Anspruch 1, dadurch gekennzeichnet, daß die Diisocyanate gewählt werden aus der Gruppe der unmodifizierten aromatischen oder aliphatischen Diisocyanate, beispielsweise 4,4'-Diisocyanatodiphenylmethan, und der durch Präpolymerisierung mit Polyolen oder Polyetherpolyolen gebildeten, modifizierten Produkte, beispielsweise des durch Präpolymerisierung mit Tripropylenglycol verflüssigten 4,4'-Diisocyanatodiphenylmethans.

5. Schäume nach Anspruch 1, dadurch gekennzeichnet, daß die Beschleuniger gewählt werden aus der Gruppe bestehend aus
- Organischen Säuren, insbesondere p-Toluolsulfonsäure, n-Butylphosphorsäure,
- Organozinnverbindungen, einschließlich deren Salze organischer und anorganischer Säuren, insbesondere Zinn-Naphthenat, Zinnbenzoat, Dibutylzinndioctoat, Dibutylzinndilaurat, Zinn-II-ethylhexoat und Dibutylzinndiacetat,
- Eisensalzen höherer Fettsäuren, insbesondere Eisenstearat,
- Isophorondiamin, Methylendianilin, Imidazolen.
- tertiären Aminen, insbesondere der Trialkylamine, wobei die Alkylreste vorteilhaft 2 - 6 Kohlenstoffatome haben.

6. Schäume nach Anspruch 1, durch folgende Zusammensetzung gekennzeichnet:
mindestens 20 Gew.-% Polyetherpolyol
* Diisocyanat in einer Menge, daß das Gewichtsverhältnis Polyetherpolyol : Diisocyanat von 15 : 1 bis 5 : 1 eingehalten wird
* 5 - 60 Gew.-% Superabsorber
* 0,001 - 1,00 Gew.-% Beschleuniger
* 0,01 - 20 Gew.-% Wasser

7. Schäume nach Anspruch 1, durch folgende Zusammensetzung gekennzeichnet:
mindestens 20 Gew.-% Polyetherpolyol
* Diisocyanat in einer Menge, daß das Gewichtsverhältnis Polyetherpolyol : Diisocyanat von 15 : 1 bis 5 : 1 eingehalten wird
* 5 - 60 Gew.-% Na-Polyacrylat
* 0,001 - 1,00 Gew.-% Beschleuniger
* 0,01 - 20 Gew.-% Wasser

8. Schäume nach Anspruch 1, durch folgende Zusammensetzung gekennzeichnet:
* 50 - 70 Gew.-% Polyetherpolyol
mindestens 5 Gew.-% Diisocyanat mit der Maßgabe, daß sich das Gewichtsverhältnis Polyetherpolyol : Diisocyanat in dem in Anspruch 1 definierten Bereich bewegt
* 15 - 35 Gew.-% Superabsorber
* 0,05 - 1,00 Gew.-% Beschleuniger
* 0,1 - 10 Gew.-% Wasser

9. Schäume nach Anspruch 1, durch folgende Zusammensetzung gekennzeichnet:
* 50 - 70 Gew.-% Polyetherpolyol
mindestens 5 Gew.-% Diisocyanat mit der Maßgabe, daß sich das Gewichtsverhältnis Polyetherpolyol : Diisocyanat in dem in Anspruch 1 definierten Bereich bewegt
* 15 - 35 Gew.-% Na-Polyacrylat
* 0,05 - 1,00 Gew.-% Beschleuniger
* 0,1 - 10 Gew.-% Wasser

10. Verfahren zur Herstellung selbstklebender hydrophiler Schäume nach einem der Ansprüche 1 - 9, dadurch gekennzeichnet, daß
- ein oder mehrere Polyetherpolyole, gewählt aus der Gruppe der durch Anlagerung von Propylenoxid und gegebenenfalls Ethylenoxid an übliche Startermoleküle gebildeten Substanzen
- ein oder mehrere Diisocyanate,
- ein Superabsorber
- ein oder mehrere Beschleuniger, gewählt aus der Gruppe der üblichen Beschleuniger für Polyurethane,
- Wasser,
vereinigt und miteinander vermischt und gegebenenfalls zusätzlich mechanisch und/oder durch Gaseinleitung zu Schäumen aufgeschlagen werden und gegebenenfalls
- zu flächigen Gegenständen ausgegossen oder ausgestrichen werden oder
- auf flächige Träger aufgebracht werden oder
- zu raumfüllenden Gegenständen gegossen werden.

11. Flächige Gegenstände, bestehend aus
- nach Anspruch 10 erhältlichen flächigen Gegenständen oder aus
- mit Schäumen nach Anspruch 1 beschichteten flächigen Trägern, letztere gewählt aus der Gruppe der Gewebe, Gewirke, Vliese und Folien oder
- nach Anspruch 10 erhältlichen beschichteten Trägern, letztere gewählt aus der Gruppe der Gewebe, Gewirke, Vliese und Folien.

12. Verwendung von Schäumen nach Anspruch 1 oder flächigen Gegenständen nach Anspruch 11 zur Herstellung von Gegenständen zur Versorgung von Wunden.

13. Verwendung von Schäumen nach Anspruch 9 in Form flächiger Gegenstände als kohäsive Binden.

## Claims

1. Hydrophilic foams based on a polyurethane gel material which can be obtained from
- one or more polyether-polyols selected from the group comprising substances formed by the addition of propylene oxide and optionally ethylene oxide on to conventional starter molecules
- one or more diisocyanates,
- one or more accelerators selected from the group comprising conventional accelerators for polyurethanes,
- the weight ratio of polyether-polyol : diisocyanate being selected in the range from 15 : 1 to 5 : 1,
- one or more superabsorbents, these superabsorbents being selected from the group comprising the sodium salts of polyacrylates or from the group comprising carboxymethyl cellulose derivatives, karaya, cellulose, pectin, polyvinyl alcohol, polyvinylpyrrolidone and gelatin
- water,
- and optionally conventional auxiliary substances and/or additives.

2. Foams according to Claim 1, characterised in that the ratio of polyether-polyol : diisocyanate is selected in the range from 12 : 1 to 8 : 1.

3. Foams according to Claim 1, characterised in that the polyether-polyols are selected from those prepared with pentaerythritol, sorbitol, trimethylolpropane or ethylenediamine as starter molecules.

4. Foams according to Claim 1, characterised in that the diisocyanates are selected from the group comprising unmodified aromatic or aliphatic diisocyanates, for example 4,4' -diisocyanatodiphenylmethane, and modified products formed by prepolymerisation with polyols or polyether-polyols, for example 4,4'-diisocyanatodiphenylmethane liquefied by prepolymerisation with tripropylene glycol.

5. Foams according to Claim 1, characterised in that the accelerators are selected from the group comprising
- organic acids, especially p-toluenesulphonic acid and n-butylphosphoric acid,
- organotin compounds, including their salts with organic and inorganic acids, especially tin naphthenate, tin benzoate, dibutyltin dioctoate, dibutyltin dilaurate, tin(II) ethylhexanoate and dibutyltin diacetate,
- iron salts of higher fatty acids, especially iron stearate,
- isophoronediamine, methylenedianiline and imidazoles.
- tertiary amines, especially trialkylamines, the alkyl radicals advantageously having 2 - 6 carbon atoms.

6. Foams according to Claim 1, characterised in that they have the following composition:
at least 20 % by weight of polyether-polyol
* diisocyanate in an amount such that the weight ratio of polyether-polyol: diisocyanate is kept at from 15:1 to 5:1
* 5 - 60 % by weight of superabsorbent
* 0.001 - 1.00 % by weight of accelerator
* 0.01 - 20 % by weight of water

7. Foams according to Claim 1, characterised in that they have the following composition:
at least 20 % by weight of polyether-polyol
* diisocyanate in an amount such that the weight ratio of polyether-polyol: diisocyanate is kept at from 15:1 to 5:1
* 5 - 60 % by weight of sodium polyacrylate
* 0.001 - 1.00 % by weight of accelerator
* 0.01 - 20 % by weight of water

8. Foams according to Claim 1, characterised in that they have the following composition:
* 50 - 70 % by weight of polyether-polyol
at least 5 % by weight of diisocyanate with the proviso that the weight ratio of polyether-polyol:diisocyanate is within the range defined in Claim 1
* 15 - 35 % by weight of superabsorbent
* 0.05 - 1.00 % by weight of accelerator
* 0.1 - 10 % by weight of water

9. Foams according to Claim 1, characterised in that they have the following composition:
* 50 - 70 % by weight of polyether-polyol
at least 5 % by weight of diisocyanate with the proviso that the weight ratio of polyether-polyol:diisocyanate is within the range defined in Claim 1
* 15 - 35 % by weight of sodium polyacrylate
* 0.05 - 1.00 % by weight of accelerator
* 0.1 - 10 % by weight of water

10. A process for the manufacture of self-adhesive hydrophilic foams according to one of Claims 1 - 9, characterised in that
- one or more polyether-polyols selected from the group comprising substances formed by the addition of propylene oxide and optionally ethylene oxide on to conventional starter molecules,
- one or more diisocyanates,
- one superabsorbent,
- one or more accelerators selected from the group comprising conventional accelerators for polyurethanes, and
- water
are combined and mixed together and, if appropriate, additionally foamed, mechanically and/or by the introduction of gas, and, if appropriate,
- cast or spread out to form two-dimensional objects, or
- applied to two-dimensional bases, or
- cast to form space-filling objects.

11. Two-dimensional objects consisting of
- two-dimensional objects obtainable according to Claim 10, or
- two-dimensional bases coated with foams according to Claim 1, said bases being selected from the group comprising woven fabrics, knitted fabrics, non-woven fabrics and films, or
- coated bases obtainable according to Claim 10, said bases being selected from the group comprising woven fabrics, knitted fabrics, non-woven fabrics and films.

12. Use of foams according to Claim 1 or two-dimensional objects according to Claim 11 for the manufacture of objects for the care of wounds.

13. Use of foams according to Claim 9 in the form of two-dimensional objects as cohesive bandages.

## Revendications

1. Mousses hydrophiles à base d'une matière de type gel de polyuréthanne, pouvant être obtenues à partir
- d'un ou de plusieurs polyétherpolyols, choisis dans le groupe des substances formées par fixation par addition d'oxyde de propylène et éventuellement d'oxyde d'éthylène sur des molécules de départ usuelles,
- d'un ou de plusieurs diisocyanates,
- d'un ou de plusieurs accélérateurs, choisis dans le groupe des accélérateurs usuels pour polyuréthannes,
- le rapport pondéral polyétherpolyol:diisocyanate étant choisi dans la plage de 15:1 à 5:1,
- d'un ou de plusieurs superabsorbants, ces superabsorbants étant choisis dans le groupe des sels sodiques des polyacrylates ou dans le groupe constitué par les dérivés de carboxyméthylcellulose, le karaya, la cellulose, la pectine, le poly(alcool vinylique), la polyvinylpyrrolidone et la gélatine,
- d'eau,
- ainsi qu'éventuellement d'additifs et/ou adjuvants usuels,

2. Mousses selon la revendication 1, caractérisées en ce que le rapport polyétherpolyol:diisocyanate est choisi dans la plage allant de 12:1 à 8:1.

3. Mousses selon la revendication 1, caractérisées en ce que les polyétherpolyols sont choisis parmi ceux préparés avec du pentaérythritol, du sorbitol, du triméthylolpropane ou de l'éthylènediamine en tant que molécules de départ.

4. Mousses selon la revendication 1, caractérisées en ce que les diisocyanates sont choisis dans le groupe des diisocyanates aliphatiques ou aromatiques non modifiés, par exemple le 4,4'-diisocyanatodiphényléthane, et des produits modifiés, formés par prépolymérisation avec des polyols ou des polyétherpolyols, par exemple le 4,4'-diisocyanatodiphénylméthane liquéfié olymérisation avec du tripropylèneglycol.

5. Mousses selon la revendication 1, caractérisées en ce que les accélérateurs sont choisis dans le groupe constitué par
- des acides organiques, notamment l'acide p-toluène-sulfonique, l'acide n-butylphosphorique,
- des composés organostanniques, y compris leurs sels d'acides organiques ou minéraux, en particulier le naphténate d'étain, le benzoate d'étain, le dioctoate de dibutylétain, le dilaurate de dibutylétain, l'éthylhexanoate stanneux et le diacétate de dibutylétain,
- des sels de fer d'acides gras supérieurs notamment le stéarate de fer,
- l'isophoronediamine, la méthylènedianiline, les imidazoles,
- les amines tertiaires, en particulier les trialkylamines, les radicaux alkyle ayant avantageusement de 2 à 6 atomes de carbone.

6. Mousses selon la revendication 1, caractérisées par la composition suivante:
- au moins 20% en poids de polyétherpolyol un diisocyanate en une quantité telle que le rapport pondéral polyétherpolyol:diisocyanate de 15:1 à 5:1 soit maintenu
- 5 - 60% en poids de superabsorbant
- 0,001 - 1,00% en poids d'accélérateur
- 0,01 - 20% en poids d'eau

7. Mousses selon la revendication 1, caractérisées par la composition suivante:
- au moins 20% en poids de polyétherpolyol un diisocyanate en une quantité telle que le rapport pondéral polyétherpolyol:diisocyanate de 15:1 à 5:1 soit maintenu
- 5 - 60% en poids de polyacrylate de Na
- 0,001 - 1,00% en poids d'accélérateur
- 0,01 - 20% en poids d'eau

8. Mousses selon la revendication 1, caractérisées par la composition suivante:
- 50 - 70% en poids de polyétherpolyol
- au moins 5% en poids de diisocyanate étant entendu que le rapport pondéral polyétherpolyol: diisocyanate s'échelonne dans la plage définie à la revendication 1
- 15 - 35% en poids de superabsorbant
- 0,05 - 1,00% en poids d'accélérateur
- 0,1 - 10% en poids d'eau

9. Mousses selon la revendication 1, caractérisées par la composition suivante:
- 50 - 70% en poids de polyétherpolyol
- au moins 5% en poids de diisocyanate étant entendu que le rapport pondéral polyétherpolyol: diisocyanate s'échelonne dans la plage définie à la revendication 1
- 15 - 35% en poids de polyacrylate de Na
- 0,05 - 1,00% en poids d'accélérateur
- 0,1 - 10% en poids d'eau

10. Procédé pour la fabrication de mousses hydrophiles autocollantes selon l'une des revendications 1 à 9, caractérisé en ce que l'on réunit et mélange les uns avec les autres
- un ou plusieurs polyétherpolyols, choisis dans le groupe des substances formées par fixation par addition d'oxyde de propylène et éventuellement d'oxyde d'éthylène sur des molécules usuelles de produits de départ,
- un ou plusieurs diisocyanates,
- un superabsorbant,
- un ou plusieurs accélérateurs, choisis dans le groupe des accélérateurs usuels pour polyuréthannes,
- de l'eau,
et éventuellement on les bat en plus en mousse, mécaniquement et/ou par introduction d'un gaz, et éventuellement
- on les coule ou on les étale en articles plans ou
- on les applique sur des supports plans ou
- on les coule en articles tridimensionnels.

11. Articles plans, constitués
- d'articles plans pouvant être obtenus selon la revendication 10 ou
- de supports plans revêtus avec des mousses selon la revendication 1, ces derniers étant choisis dans le groupe des tissus, tricots, non-tissés et pellicules ou
de supports revêtus pouvant être obtenus selon la revendication 10, ces derniers étant choisis dans le groupe des tissus, tricots, non-tissés et pellicules.

12. Utilisation de mousses selon la revendication 1 ou d'articles plans selon la revendication 11, pour la fabrication d'articles destinés au soin de plaies.

13. Utilisation de mousses selon la revendication 9, sous forme d'articles plans, en tant que bandages cohésifs.
